# EUROPEAN PATENT APPLICATION

(11) **EP 4 442 188 A1**
(43) Date of publication of application: **09.10.2024**
(21) Application number: 21966393.7
(22) Date of filing: 02.12.2021
(51) Int. Cl.: A61B 1/045, A61B 1/00

(54) **ENDOSCOPIC EXAMINATION ASSISTANCE DEVICE, ENDOSCOPIC EXAMINATION ASSISTANCE SYSTEM, ENDOSCOPIC EXAMINATION ASSISTANCE METHOD, AND RECORDING MEDIUM**

(71) Applicant: NEC Corporation, 108-8001 Tokyo (JP)
(72) Inventor: KAMIJO Kenichi, Tokyo 108-8001 (JP); TAKAHASHI Ikuma, Tokyo 108-8001 (JP); OKUTSU Motoyasu, Tokyo 108-8001 (JP); OHTSUKA Shota, Tokyo 108-8001 (JP); KIMURA Tatsu, Tokyo 108-8001 (JP)
(74) Representative: Betten & Resch
(86) International application number: PCT/JP2021/044222
(87) International publication number: WO 2023/100310

(57) **Abstract**

Provided is a technology for reducing the likelihood of oversight of lesions during endoscopic examination. An endoscopic examination assistance device (30b) comprises: an acquisition unit (31b) which acquires an endoscopic image captured by an imaging unit of an endoscope and imaging unit position information indicating the position of the imaging unit of the endoscope within a lumen at the time when the endoscopic image is captured; a lesion detection unit (32b) which detects a lesion from the endoscopic image; a determination unit (33b) which determines whether the step being performed is an insertion step for inserting the endoscope to a turnaround point in the lumen or a drawing step for drawing out the endoscope from the turnaround point in the lumen; and a notification control unit (34b) which, in the case where the determination result indicates the drawing step, causes a notification device to give notification according to the positional relationship between the imaging unit position information and lesion position information indicating the position of the lesion detected in the insertion step.

## Description

### Technical Field

The present invention relates to an endoscopic examination assistance device, an endoscopic examination assistance system, an endoscopic examination assistance method, and a recording medium.

### Background Art

In the endoscopic examination, the scope is inserted deep while washing away dirt adhering to an organ, and then removed while observing the inside of the organ.

PTL 1 describes that it is determined whether an inspection step is an insertion step or a removal step, and a detection result of a region of interest is notified according to the determined step.

### Citation List

### Patent Literature

PTL 1: WO 2020/017212 A1

### Summary of Invention

### Technical Problem

Insertion of an endoscope is technically difficult, and a doctor is required to concentrate on insertion. Therefore, it is difficult for a doctor to confirm the detection result of a lesion at the time of insertion, and the doctor may overlook the lesion only by the confirmation at the time of removal. The present invention has been made in view of the above problem, and an object thereof is to reduce the possibility of overlooking a lesion.

### Solution to Problem

An endoscopic examination assistance device according to an aspect of the present invention including an acquisition means for acquiring an endoscopic image captured by an imaging unit of an endoscope and imaging unit position information indicating a position of the imaging unit of the endoscope within a lumen at a time when the endoscopic image is captured, a lesion detection means for detecting a lesion from the endoscopic image, a determination means for determining whether it is an insertion step for inserting the endoscope to a turnaround point in the lumen or a removal step for removing the endoscope from a turnaround point in the lumen, and a notification control means for, in a case where the determination result indicates the removal step, causing a notification device to give notification according to a positional relationship between the imaging unit position information and lesion position information indicating a position of a lesion detected in the insertion step.

An endoscopic examination assistance system according to an aspect of the present invention includes an endoscopic examination assistance device, and a notification device, in which the endoscopic examination assistance device includes an acquisition means for acquiring an endoscopic image captured by an imaging unit of an endoscope and imaging unit position information indicating a position of the imaging unit of the endoscope within a lumen at a time when the endoscopic image is captured, a lesion detection means for detecting a lesion from the endoscopic image, a determination means for determining whether it is an insertion step for inserting the endoscope to a turnaround point in the lumen or a removal step for removing the endoscope from a turnaround point in the lumen, and a notification control means for, in a case where the determination result indicates the removal step, causing the notification device to give notification according to a positional relationship between the imaging unit position information and lesion position information indicating a position of a lesion detected in the insertion step.

An endoscopic examination assistance method according to an aspect of the present invention causes one or a plurality of endoscopic examination assistance devices to execute acquiring an endoscopic image captured by an imaging unit of an endoscope and imaging unit position information indicating a position of the imaging unit of the endoscope within a lumen at a time when the endoscopic image is captured, detecting a lesion from the endoscopic image, determining whether it is an insertion step for inserting the endoscope to a turnaround point in the lumen or a removal step for removing the endoscope from a turnaround point in the lumen, and in a case where the determination result indicates the removal step, causing a notification device to give notification according to a positional relationship between the imaging unit position information and lesion position information indicating a position of a lesion detected in the insertion step.

A recording medium according to an aspect of the present invention has stored therein a program causing a computer to function as an acquisition means for acquiring an endoscopic image captured by an imaging unit of an endoscope and imaging unit position information indicating a position of the imaging unit of the endoscope within a lumen at a time when the endoscopic image is captured, a lesion detection means for detecting a lesion from the endoscopic image, a determination means for determining whether it is an insertion step for inserting the endoscope to a turnaround point in the lumen or a removal step for removing the endoscope from a turnaround point in the lumen, and a notification control means for, in a case where the determination result indicates the removal step, causing a notification device to give notification according to a positional relationship between the imaging unit position information and lesion position information indicating a position of a lesion detected in the insertion step.

### Advantageous Effects of Invention

According to an aspect of the present invention, it is possible to reduce the possibility of overlooking a lesion in an endoscopic examination.

### Brief Description of Drawings

[Fig. 1] Fig. 1 is a block diagram illustrating an example of an overall configuration of an endoscopic examination assistance system according to a first example embodiment of the present invention.
[Fig. 2] Fig. 2 is a block diagram illustrating a configuration of an endoscopic examination assistance device according to the first example embodiment of the present invention.
[Fig. 3] Fig. 3 is a view schematically illustrating an example of a step of inserting and removing an endoscope.
[Fig. 4] Fig. 4 is a view schematically illustrating an example of a screen output to a display device.
[Fig. 5] Fig. 5 is a view schematically illustrating an example of a screen output to the display device.
[Fig. 6] Fig. 6 is a flowchart illustrating an operation of the endoscopic examination assistance device according to the first example embodiment of the present invention.
[Fig. 7] Fig. 7 is a block diagram illustrating a configuration of an endoscopic examination assistance device according to a second example embodiment of the present invention.
[Fig. 8] Fig. 8 is a flowchart illustrating an operation of the endoscopic examination assistance device according to the second example embodiment of the present invention.
[Fig. 9] Fig. 9 is a block diagram illustrating a configuration of an endoscopic examination assistance device according to a third example embodiment of the present invention.
[Fig. 10] Fig. 10 is a view illustrating an example of a hardware configuration of the endoscopic examination assistance devices according to the first, second, and third example embodiments of the present invention.

### Example Embodiment

Example embodiments of the present invention will be described with reference to the drawings.

### (Endoscopic examination assistance system 1)

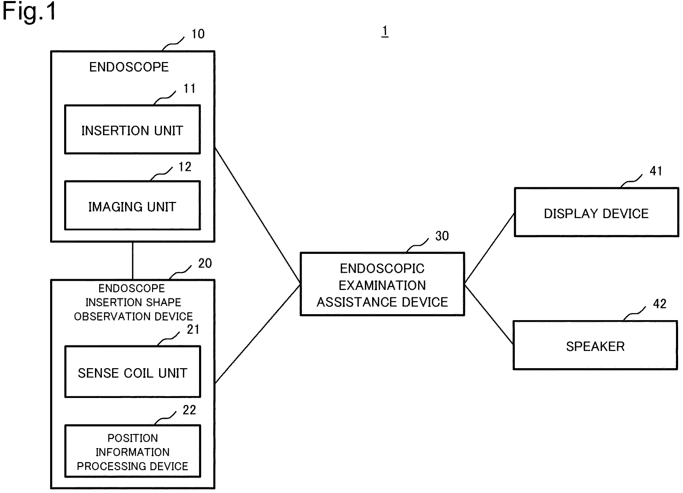

An example of a configuration of an endoscopic examination assistance system 1 to which an endoscopic examination assistance device 30 according to a first example embodiment to be described later is applied will be described with reference to Fig. 1. Fig. 1 is a view schematically illustrating an example of a configuration of a system. The endoscopic examination assistance system 1 is a system that supports endoscopic examination when a doctor performs the endoscopic examination on a subject.

As illustrated in Fig. 1, the endoscopic examination assistance system 1 includes an endoscope 10, an endoscope insertion shape observation device 20, an endoscopic examination assistance device 30, a display device 41, and a speaker 42. Each block may be mounted on the same terminal in an arbitrary combination.

The endoscope 10 is a portion to be inserted into a celom of the subject, and includes an insertion unit 11 that is bendable and tubular, and the insertion unit 11 includes an imaging unit 12 that images the inside of the lumen at a distal end thereof. Here, the lumen refers to a space inside a tubular organ, and for example, spaces inside the large intestine, the small intestine, the esophagus, and the stomach are also lumens. The endoscopic image captured by the imaging unit 12 is transmitted to the endoscopic examination assistance device 30 via a cable inside the insertion unit 11. Hereinafter, the endoscopic examination of the large intestine will be described as an example, but the endoscopic examination of other sites can be similarly performed.

Here, source coils (not illustrated) are provided at a plurality of places of the insertion unit 11. For example, the source coils are arranged at predetermined intervals throughout the insertion unit 11. The source coil generates a magnetic field by a drive signal from the endoscope insertion shape observation device 20. Therefore, the endoscope 10 may be any endoscope as long as magnetic coils for specifying the shape are built in a plurality of places of the insertion unit 11. Since a known configuration can be used as other configurations of the endoscope 10, illustration and description of other configurations are omitted. Alternatively, an instrument in which a coil is built as a magnetic coil (not built in the endoscope) can be used in a state of being inserted into the endoscope 10.

The endoscope insertion shape observation device 20 is a device that outputs position information (hereinafter, referred to as imaging unit position information) indicating the position of the imaging unit 12 in the lumen, and includes at least a sense coil unit 21 and a position information processing device 22. The sense coil unit 21 is a unit that detects a magnetic field generated from a plurality of source coils provided in the insertion unit 11 of the endoscope 10.

First, the position information processing device 22 obtains the shape of the insertion unit 11 inserted into the large intestine based on the magnetic field detected by the sense coil unit 21. For example, the position information processing device 22 calculates three-dimensional coordinates of each source coil based on the magnetic field detected by the sense coil unit 21. Since the source coil is provided at a predetermined position of the insertion unit 11, it can be said that these three-dimensional coordinates indicate the shape of the insertion unit 11.

Then, the position information processing device 22 generates imaging unit position information that is position information indicating the position of the imaging unit 12 in the large intestine from the shape of the insertion unit 11 and the general shape of the large intestine.

The imaging unit position information may be three-dimensional coordinates indicating the position of the imaging unit 12, coordinates obtained by two-dimensionally projecting the three-dimensional coordinates, or image data obtained by imaging a projection result. The imaging unit position information may be information indicating where the imaging unit 12 is located in each site in the large intestine, the information being estimated based on the coordinate information. In this case, the imaging unit position information includes, for example, any of the cecum, the ascending colon, the transverse colon, the descending colon, the sigmoid colon, and the rectum.

The imaging unit position information indicating the site where the imaging unit 12 is located can also be generated in a lumen other than the large intestine. That is, the imaging unit position information may be information indicating a site in the lumen in which the imaging unit 12 is located, the information being generated from the shape of the endoscope 10 in the lumen, the shape of the lumen, and the lumen model indicating each site constituting the lumen. The site in the lumen where the imaging unit 12 is located can also be determined by analyzing an endoscopic image captured by the imaging unit 12. For example, by using a machine-learned model constructed by machine learning using teacher data in which a name or the like of a site is labeled as correct answer data in an endoscopic image captured at each site, it is possible to determine which site is included in the captured endoscopic image.

The position information processing device 22 outputs the imaging unit position information generated as described above to the endoscopic examination assistance device 30. Since other configurations of the endoscope insertion shape observation device 20 can be known, illustration and description of other configurations are omitted.

The endoscopic examination assistance device 30 is a device that supports endoscopic examination by the endoscope 10, and is connected to the endoscope 10, the endoscope insertion shape observation device 20, the display device 41, and the speaker 42. The endoscopic examination assistance device 30 acquires an endoscopic image from the endoscope 10 and acquires imaging unit position information from the endoscope insertion shape observation device 20.

The endoscopic examination assistance device 30 causes the display device 41 and the speaker 42 to give notification according to the positional relationship between the imaging unit position information and the position information (hereinafter, referred to as lesion position information) indicating the position of the lesion detected in the insertion step based on the acquired endoscopic image and the imaging unit position information. A specific method for giving the notification will be described later. Then, the endoscopic examination assistance device 30 causes the display device 41 to output the image of the detected lesion, the lesion position information, and the determined step in association with each other. The determined step is an insertion step for inserting the endoscope 10 or a removal step for removing the endoscope 10, and these processes are determined by the endoscopic examination assistance device 30. A specific method for outputting will be described later.

The display device 41 displays the display information received from the endoscopic examination assistance device 30 on a screen. The speaker 42 outputs a voice received from the endoscopic examination assistance device 30. These devices are examples of notification devices to be notified as described above by the endoscopic examination assistance device 30. The notification device is not limited to the display device 41 and the speaker 42 as long as the notification device outputs information to be notified in such a manner that the target user can recognize the information. For example, in a case where the endoscopic examination assistance device 30 includes a display device, the display device may be used as a notification device.

### [First example embodiment]

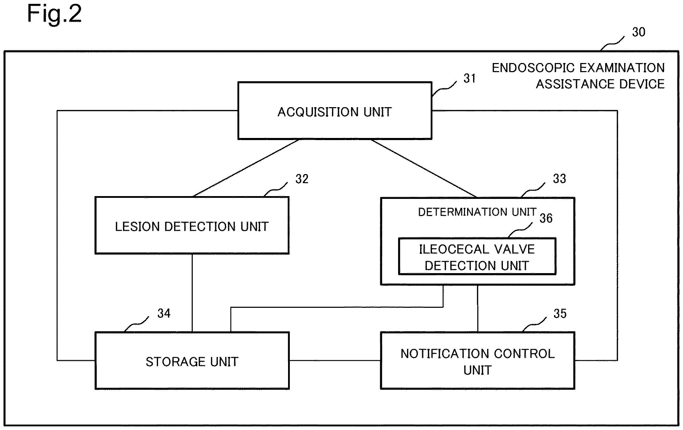

A first example embodiment will be described with reference to Figs. 2 to 4.

### (Endoscopic examination assistance device 30)

Components of the endoscopic examination assistance device 30 according to the first example embodiment will be described with reference to Fig. 2. Fig. 2 is a block diagram illustrating a configuration of the endoscopic examination assistance device 30. As illustrated in Fig. 2, the endoscopic examination assistance device 30 includes an acquisition unit 31, a lesion detection unit 32, a determination unit 33, a storage unit 34, a notification control unit 35, and an ileocecal valve detection unit 36.

The acquisition unit 31 is an acquisition means for acquiring an endoscopic image captured by the imaging unit 12 of the endoscope and imaging unit position information indicating the position of the imaging unit 12 of the endoscope in the lumen at the time of capturing the endoscopic image. The acquisition unit 31 acquires an endoscopic image captured by the imaging unit 12 from the endoscope 10, and acquires position information of the imaging unit 12 in the lumen at the time of capturing the endoscopic image, that is, imaging unit position information from the endoscope insertion shape observation device 20. It is assumed that time information such as acquired date and time and inspection elapsed time is associated with the acquired endoscopic image and imaging unit position information. The acquisition unit 31 outputs the acquired endoscopic image to the lesion detection unit 32, outputs the acquired endoscopic image and imaging unit position information to the determination unit 33, and outputs the acquired imaging unit position information to the storage unit 34 and the notification control unit 35.

The lesion detection unit 32 is a lesion detection means for detecting a lesion from an endoscopic image. The lesion detection unit 32 detects a lesion from the endoscopic image input from the acquisition unit 31. Specifically, the lesion detection unit 32 detects a lesion by inputting an endoscopic image to an identifier that has machine-learned image features of the lesion and identifying the lesion. The lesion here is not limited to a lesion caused by a disease, and includes a lesion in a state different from a normal state in appearance. Examples of the lesion include treatment marks such as polyps, cancer, large intestine diverticula, inflammation, endoscopic mucosal resection (EMR) scars or endoscopic submucosal dissection (ESD) scars, clip locations, bleeding points, perforations, vascular dysplasia, and the like. The identifier used by the lesion detection unit 32 is not limited to one generated by machine learning, and may be any identifier capable of detecting a lesion. The lesion detection unit 32 outputs lesion information on the detected lesion to the storage unit 34. It is assumed that the lesion information includes a lesion image which is an image of a detected lesion, and is associated with time information such as a detection date and time and an inspection elapsed time. The lesion information includes lesion position information indicating a position of a lesion.

The determination unit 33 is a determination means for determining whether it is an insertion step for inserting the endoscope 10 to the turnaround point in the lumen or a removal step for removing the endoscope from the turnaround point in the lumen. The determination unit 33 determines whether it is the insertion step or the removal step based on at least one of the endoscopic image and the imaging unit position information input from the acquisition unit 31. Specifically, the determination unit 33 detects the ileocecal valve from the endoscopic image by the ileocecal valve detection unit 36 included in the determination unit 33, thereby determining the process before the detection of the ileocecal valve as the insertion step and the process after the detection of the ileocecal valve as the removal step. Based on the input imaging unit position information, the determination unit 33 may determine that the insertion step is performed until the imaging unit position information reaches the turnaround point, and the removal step is performed after the imaging unit position information reaches the turnaround point, or may use both the determination by the endoscopic image and the determination by the imaging unit position information. The determination unit 33 outputs the determination result to the notification control unit 35. The determination unit 33 outputs time information such as the determined date and time and the inspection elapsed time to the storage unit 34 in association with the determination result. The determination unit 33 may omit the process of determining whether the process is the insertion step or the removal step and output the determination result as the removal step during the same inspection after the process is once determined as the removal step. In that case, the determination unit 33 can reduce the calculation cost for the processing.

The storage unit 34 stores the imaging unit position information input from the acquisition unit 31, the lesion information input from the lesion detection unit 32, and the determination result input from the determination unit 33. The storage unit 34 stores the input imaging unit position information, lesion information, and determination result in association with each other based on the time information. Specifically, in the storage unit 34, the imaging unit position information, the lesion information, and the determination result in which associated time information indicates the same time are stored in association. The storage unit 34 does not necessarily need to exist inside the endoscopic examination assistance device 30. In a case where the storage unit 34 exists outside the endoscopic examination assistance device 30, the storage unit 34 can access the endoscopic examination assistance device 30. The endoscopic examination assistance system including such a storage unit 34 functions similarly to the endoscopic examination assistance device 30.

In a case where the determination result of the determination unit 33 indicates the removal step, the notification control unit 35 is a notification control means for causing notification according to the positional relationship between the current imaging unit position information and the lesion position information indicating the position of the lesion detected in the insertion step. The notification control unit 35 causes the display device 41 and the speaker 42 to give notification based on the imaging unit position information input from the acquisition unit 31, the determination result input from the determination unit 33, and the lesion position information, the lesion information, and the determination result acquired from the storage unit 34. Specifically, in a case where the position indicated by the imaging unit position information input from the acquisition unit 31 and the position indicated by the lesion position information (the position information of the lesion detected in the insertion step) stored in the storage unit 34 are equal to or less than a predetermined distance, the notification control unit 35 causes notification that there is a lesion detected in the insertion step nearby the position indicated by the current position information. In a case where the position indicated by the imaging unit position information input from the acquisition unit 31 passes the position indicated by the lesion position information stored in the storage unit 34, the notification control unit 35 may cause notification that a lesion detected in the insertion step has been passed. The notification control unit 35 may cause only one of the display device 41 and the speaker 42 to give notification.

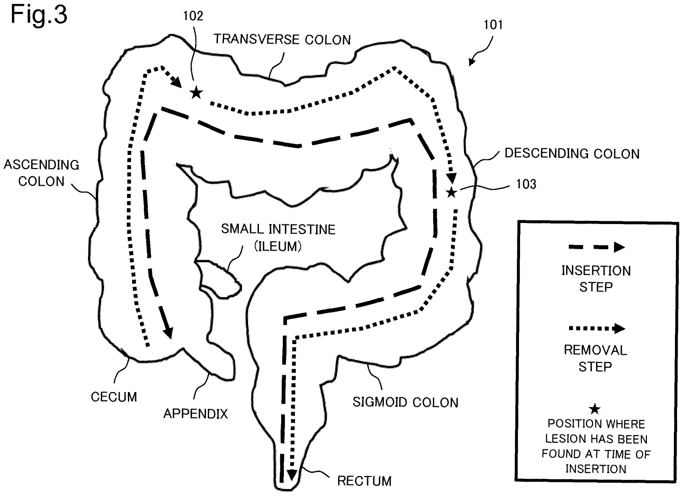

Fig. 3 is a view schematically illustrating an example of a step of inserting and removing the endoscope 10. In Fig. 3, a path of insertion and removal of the endoscope 10 in an image 101 of the large intestine is indicated by an arrow. As illustrated, the endoscope 10 is inserted from the rectum to the cecum (insertion step) and then removed (removal step). There is an ileocecal valve (not illustrated) at the junction between the cecum and the ileum. In Fig. 3, lesions 102 and 103 detected in the insertion step are indicated by stars. For these lesions, the lesion position information is stored at the time of detection.

In the removal step, in a case where the distance between the position indicated by the imaging unit position information input from the acquisition unit 31 and the position indicated by the lesion position information is equal to or less than a threshold, the notification control unit 35 causes the display device 41 and the speaker 42 to give notification that a lesion is present nearby.

For example, with respect to the lesion 102 detected in the transverse colon in the insertion step, the notification control unit 35 may cause notification that there is a lesion nearby at the timing when the position indicated by the imaging unit position information input from the acquisition unit 31 in the removal step changes from the ascending colon to the transverse colon. Similarly, even when the position indicated by the imaging unit position information is near the lesion 103, the notification control unit 35 may cause notification that there is a lesion nearby.

In the removal step, in a case where the position indicated by the imaging unit position information input from the acquisition unit 31 passes the position indicated by the lesion position information, the notification control unit 35 may cause the display device 41 and the speaker 42 to give notification that the lesion detected in the insertion step has been passed.

Specifically, for the lesion 102 detected in the transverse colon in the insertion step, the notification control unit 35 may cause notification that the lesion has been passed at the timing when the position indicated by the imaging unit position information input from the acquisition unit 31 in the removal step has moved from the transverse colon to the descending colon.

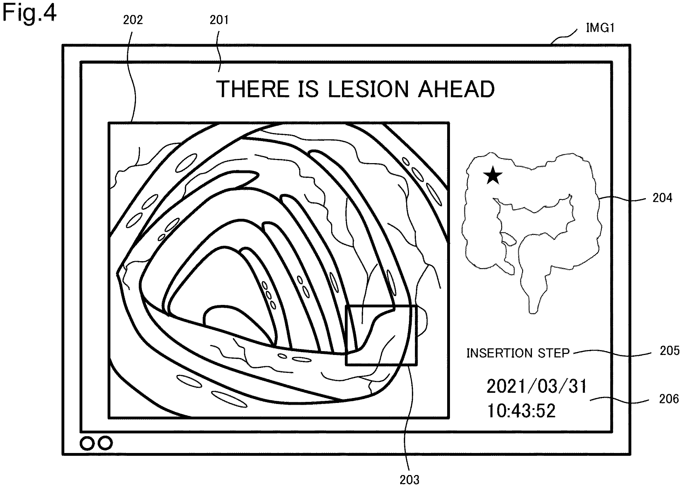

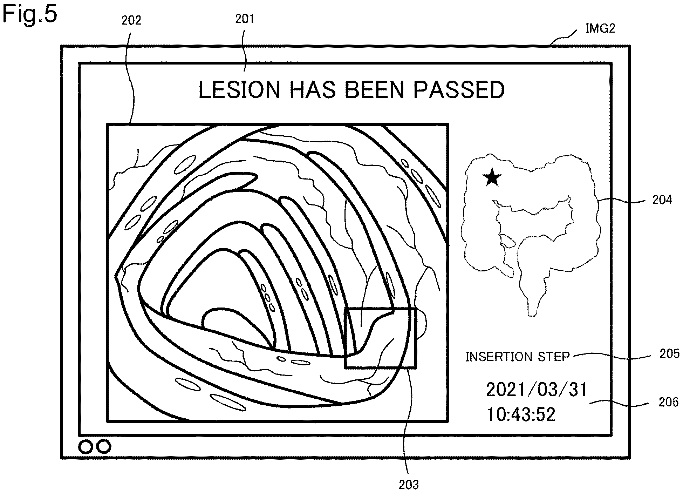

Figs. 4 and 5 are views schematically illustrating an example of a screen output to the display device 41. An image IMG1 illustrated in Fig. 4 is an example of a notification screen for notifying that there is a lesion nearby. For example, as illustrated in Fig. 4, the notification control unit 35 outputs a message 201 indicating that the lesion is ahead of the current position of the imaging unit 12, an image 202 of the detected lesion, information 203 indicating the lesion site in the image 202, information 204 indicating the position of the detected lesion in the large intestine (in this example, the position of the lesion is indicated by a star mark in a view schematically illustrating the shape of the large intestine), information 205 indicating the step at the time of detection (in this example, a quadrangular frame), and a date and time 206 when the lesion is detected on the screen. By confirming the output result on the screen, the doctor may know that there is a lesion ahead of the current position of the imaging unit 12, and may confirm detailed information of the lesion.

An image IMG2 illustrated in Fig. 5 is an example of a notification screen for notifying that a lesion has been passed. The image IMG2 is substantially similar to the image IMG1, but is different from the image IMG1 in that the message 201 is replaced with a message 301. The message 301 is a message indicating that the current position of the imaging unit 12 has already passed the position of the lesion. By confirming the image IMG2, the doctor may know that the imaging unit 12 has already passed the lesion, and may confirm detailed information of the lesion by moving the endoscope 10 to the lumen back side again.

The notification screen is not limited to the examples of Figs. 4 and 5, and may be any screen as long as the contents of the notification according to the positional relationship between the imaging unit position information and the lesion position information can be visually recognized. For example, the notification control unit 35 may display the position of the imaging unit 12 to be superimposed on the information 204 (a view schematically illustrating the shape of the large intestine) based on the imaging unit position information. By displaying such an image, the user (doctor or the like) may be made to recognize the positional relationship between the imaging unit position information and the lesion position information, and thus, in this case, the messages 201 and 301 can be omitted.

For example, the notification control unit 35 may cause the current endoscopic image (one imaged at the position indicated by the imaging unit position information) to be displayed together with the image 202 of the lesion. As a result, the user (doctor or the like) may search for a lesion from the current endoscopic image while comparing the current endoscopic image together with the image 202 of the lesion.

Moreover, for example, in a case where the distance between the position indicated by the imaging unit position information and the position indicated by the lesion position information is equal to or less than a predetermined distance, the notification control unit 35 may notify that there is a lesion detected in the insertion step near the position indicated by the imaging unit position information. For example, the notification control unit 35 may cause a message such as "There is a lesion detected in the insertion step nearby" to be displayed, or may highlight the lesion in the image 202 of the lesion. The method of highlighting is not particularly limited, and for example, highlighting may be performed by blinking an image (in the examples of Figs. 4 and 5, a star mark is used) indicating a lesion, changing a color, or the like.

### (Operation of endoscopic examination assistance device 30)

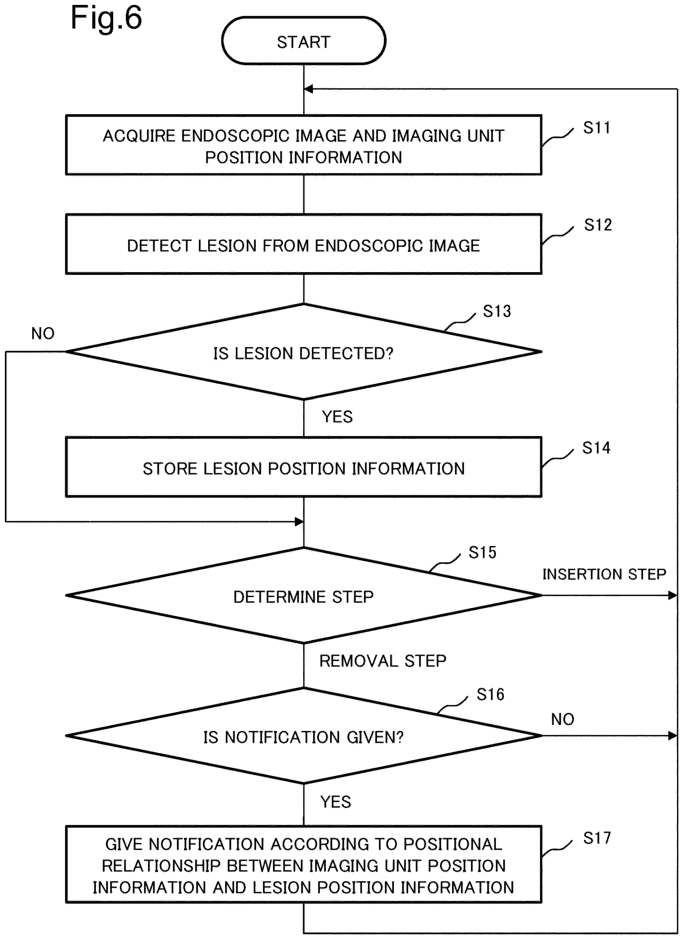

The operation of the endoscopic examination assistance device 30 according to the first example embodiment will be described with reference to Fig. 6. Fig. 6 is a flowchart illustrating a flow of processing (endoscopic examination assistance method) executed by each unit of the endoscopic examination assistance device 30.

As illustrated in Fig. 6, the acquisition unit 31 acquires an endoscopic image captured by the imaging unit 12 of the endoscope 10 and position information of the imaging unit 12 of the endoscope 10 at the time of capturing, that is, imaging unit position information (S11). Next, the lesion detection unit 32 detects a lesion from the endoscopic image acquired in S11 (S12).

In a case where a lesion is detected in the process of S12 (YES in S13), the lesion detection unit 32 stores lesion position information, which is position information of the detected lesion, in the storage unit 34 (S14). On the other hand, in a case where no lesion is detected in the process of S12 (NO in S13), the process proceeds to the process of S15.

In S15, the determination unit 33 determines whether it is the insertion step or the removal step. In a case where the determination result of the determination unit 33 in S15 indicates the insertion step, the process returns to S11. In a case where the determination result of the determination unit 33 indicates the removal step, the process proceeds to S16.

In S16, the notification control unit 35 determines whether to give notification. Here, the notification control unit 35 determines to give notification in a case where a predetermined condition is satisfied, such as a case where the distance between the position indicated by the imaging unit position information and the position indicated by the lesion position information is equal to or less than a predetermined distance. In a case where it is determined in S16 that the notification is given (YES in S16), the process proceeds to S17, and in a case where it is determined that the notification is not given (NO in S16), the process returns to S11.

In S17, the notification control unit 35 causes the notification device to give notification according to the positional relationship between the imaging unit position information in the removal step and the lesion position information of the lesion detected in the insertion step (S17). Thereafter, the process returns to S11.

As described above, according to the endoscopic examination assistance method of the present example embodiment, since the notification according to the positional relationship between the lesion position information of the lesion detected in the insertion step and the imaging unit position information in the removal step is performed, the possibility of overlooking a lesion can be reduced as compared with the case where the notification is not given.

### [Second example embodiment]

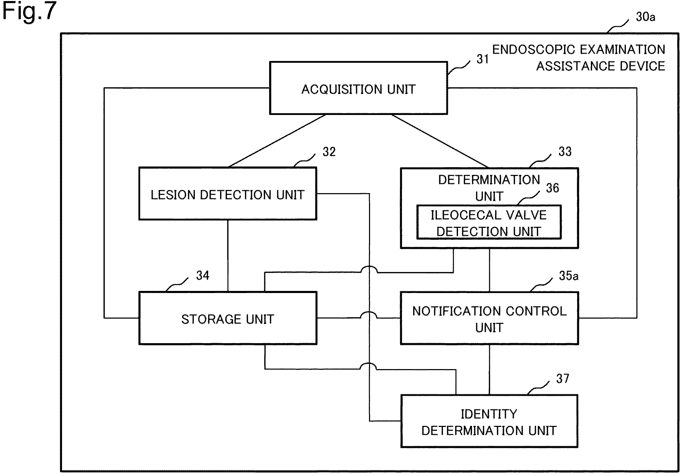

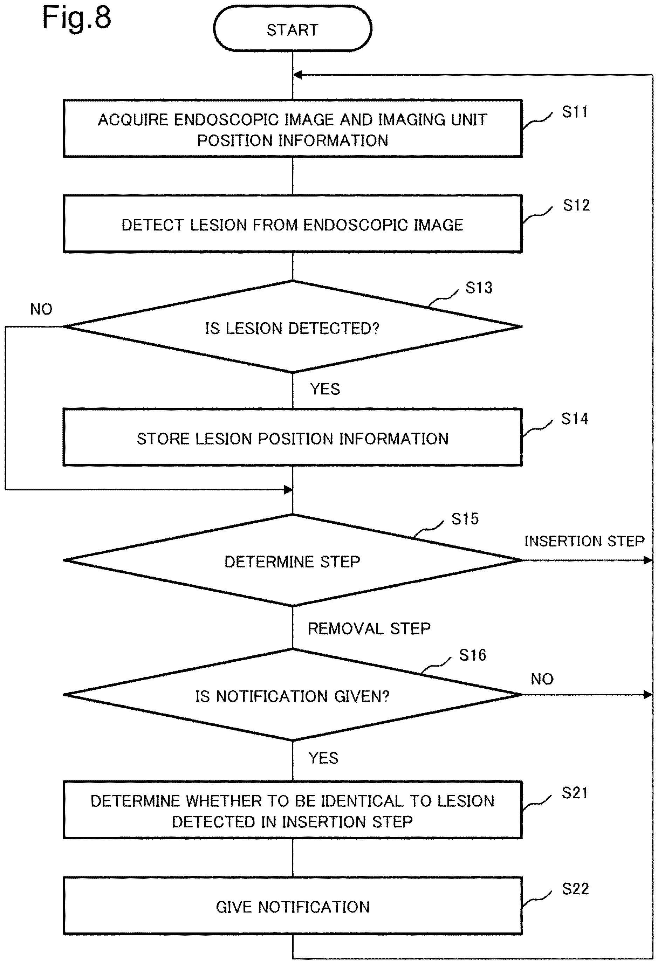

A second example embodiment will be described with reference to Figs. 7 and 8. In the second example embodiment, in addition to the operation in the first example embodiment, it is possible to control the notification based on the determination as to whether the lesion detected in the removal step is identical to the lesion detected in the insertion step.

### (Endoscopic examination assistance device 30a)

With reference to Fig. 7, components of an endoscopic examination assistance device 30a according to the second example embodiment will be described. In the configuration of the endoscopic examination assistance device 30a, a configuration that performs the same processing operation as the configuration of the endoscopic examination assistance device 30 of the first example embodiment is denoted by the same reference numeral as that in Fig. 2, and a detailed description thereof is omitted. Fig. 7 is a block diagram illustrating the configuration of the endoscopic examination assistance device 30a. As illustrated in Fig. 7, the endoscopic examination assistance device 30a includes an acquisition unit 31, a lesion detection unit 32, a determination unit 33, a storage unit 34, a notification control unit 35a, an ileocecal valve detection unit 36, and an identity determination unit 37.

The identity determination unit 37 is an identity determination means for determining whether the lesion detected in the removal step is identical to the lesion detected in the insertion step. The identity determination unit 37 determines whether the lesion detected in the removal step is identical to the lesion detected in the insertion step based on the lesion information in the removal step acquired from the lesion detection unit 32 and the lesion information in the insertion step acquired from the storage unit 34. Specifically, the identity determination unit 37 determines whether the lesion in the lesion image included in the lesion information in the removal step acquired from the lesion detection unit 32 is identical to the lesion in the lesion image included in the lesion information in the insertion step acquired from the storage unit 34. The determination on identity is made by an existing determiner that determines the identity of the images. The identity determination unit 37 may determine that the same lesion is present when the detected lesion types are identical. The identity determination unit 37 outputs an identity determination result indicating whether they are the same to the notification control unit 35a.

In a case where the determination result indicates the removal step, the notification control unit 35a is a notification control means for causing the notification device to give notification according to the positional relationship between the imaging unit position information and the lesion position information and the result of the identity determination. The notification control unit 35a causes the display device 41 and the speaker 42 to give notification based on the imaging unit position information input from the acquisition unit 31, the determination result as to whether it is the insertion step or the removal step input from the determination unit 33, the lesion position information, the lesion information, and the determination result as to whether it is the insertion step or the removal step when the lesion is detected acquired from the storage unit 34, and the identity determination result input from the identity determination unit 37. The notification control unit 35a causes the notification device to notify the notification of contents corresponding to the determination result as to whether the lesion detected in the removal step is identical to the lesion detected in the insertion step. For example, in a case where the lesion detected in the removal step is identical the lesion detected in the insertion step, the notification control unit 35a causes the notification device to give notification that the lesion is identical to the lesion detected in the insertion step. In a case where the lesion detected in the removal step is not identical to the lesion detected in the insertion step, the notification control unit 35a causes the notification device to give notification that the lesion is not identical to the lesion detected in the insertion step.

For example, in a case where the determination result input from the determination unit 33 indicates the removal step and the determination result indicating the same is input from the identity determination unit 37, the notification control unit 35a may cause the display device 41 and the speaker 42 to notify that the lesion detected in the insertion step has been detected again. In this case, when the doctor searches for the lesion detected in the insertion step, it is possible to efficiently grasp the examination situation. In a case where the current determination result input from the determination unit 33 indicates the removal step, the determination result indicating the same is not input from the identity determination unit 37, and the position indicated by the imaging unit position information input from the acquisition unit 31 has passed the position indicated by the lesion position information stored in the storage unit 34, the notification control unit 35a may cause the display device 41 and the speaker 42 to notify that the lesion detected in the insertion step has been passed without being detected. In this case, even when there is another lesion not detected in the insertion step at a position close to the lesion detected in the insertion step, and only another lesion not detected in the insertion step is detected and passed in the removal step, it is notified that the lesion detected in the insertion step is not detected. Therefore, the doctor may return (move in the insertion direction) the endoscope 10 to search for the lesion that has been passed, and the possibility of overlooking a lesion can be reduced. The notification control unit 35a may cause only one of the display device 41 and the speaker 42 to give notification.

### (Operation of endoscopic examination assistance device 30a)

The operation of the endoscopic examination assistance device 30a according to the second example embodiment will be described with reference to Fig. 8. Fig. 8 is a flowchart illustrating a flow of processing (endoscopic examination assistance method) executed by each unit of the endoscopic examination assistance device 30a. The processes of S11 to S16 in Fig. 8 are similar to the processes of the steps with the same number in Fig. 6.

As illustrated in Fig. 8, the acquisition unit 31 acquires an endoscopic image captured by the imaging unit 12 of the endoscope 10 and position information of the imaging unit 12 of the endoscope 10 at the time of capturing, that is, imaging unit position information (S11). Next, the lesion detection unit 32 detects a lesion from the endoscopic image acquired in S11 (S12). In a case where a lesion is detected by the lesion detection unit 32 (YES in S13), the lesion detection unit 32 stores the lesion position information, which is position information of the detected lesion, in the storage unit 34 (S14). Next, the determination unit 33 determines whether it is the insertion step or the removal step (S1 5). In a case where the determination result of the determination unit 33 indicates the insertion step, the process returns to S11.

In a case where the determination result of the determination unit 33 in S15 indicates the removal step and the notification is given (YES in S16), the identity determination unit 37 determines whether the lesion detected in S13 is identical to the lesion detected in the insertion step (S21). Then, the notification control unit 35a causes the notification device (for example, one or both of the display device 41 and the speaker 42) to notify the contents according to the result of the determination as to whether they are identical in S21 (S22). Thereafter, the process returns to S11.

As described above, according to the endoscopic examination assistance method executed by the endoscopic examination assistance device 30a of the present example embodiment, since the notification of the contents according to the determination result as to whether the lesion detected in the insertion step and the lesion detected in the removal step are identical is performed, it is possible to efficiently grasp the examination situation.

### [Third example embodiment]

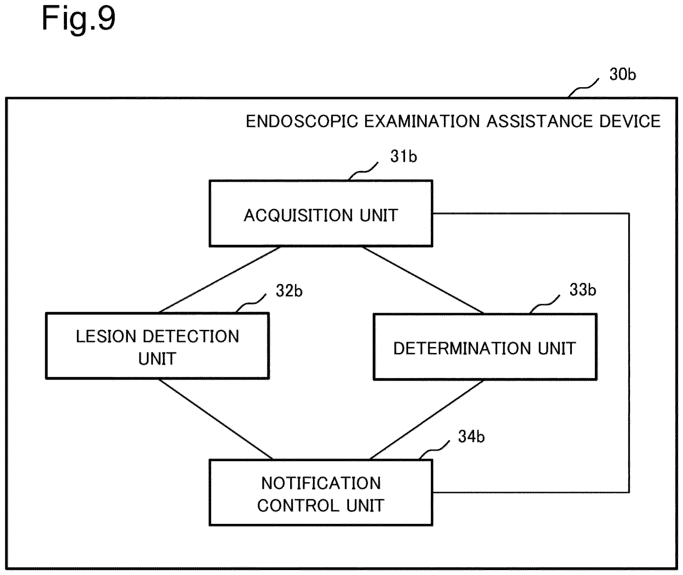

A third example embodiment will be described with reference to Fig. 9.

### (Endoscopic examination assistance device 30b)

Components of an endoscopic examination assistance device 30b according to the third example embodiment will be described with reference to Fig. 9. The third example embodiment is a basic example embodiment of the first example embodiment and the second example embodiment. The endoscopic examination assistance device 30b illustrated in Fig. 9 includes an acquisition unit 31b, a lesion detection unit 32b, a determination unit 33b, and a notification control unit 34b. For example, the acquisition unit 31b, the lesion detection unit 32b, the determination unit 33b, and the notification control unit 34b can be configured similarly to the acquisition unit 31, the lesion detection unit 32, the determination unit 33, and the notification control unit 35, respectively, but are not limited thereto.

The acquisition unit 31b acquires an endoscopic image captured by the imaging unit 12 of the endoscope 10 and imaging unit position information that is position information of the imaging unit 12 of the endoscope 10 in the lumen at the time of capturing the endoscopic image, and outputs the acquired information to the lesion detection unit 32b, the determination unit 33b, and the notification control unit 34b. The lesion detection unit 32b detects a lesion from the endoscopic image and outputs the detected lesion to the notification control unit 34b. The determination unit 33b determines whether it is an insertion step for inserting the endoscope 10 to the turnaround point in the lumen or a removal step for removing the endoscope from the turnaround point in the lumen, and outputs a determination result to the notification control unit 34b. In a case where the determination result indicates the removal step, the notification control unit 34b causes an arbitrary notification device to give notification according to the positional relationship between the imaging unit position information and the lesion position information.

According to the endoscopic examination assistance device 30b of the third example embodiment, it is possible to reduce the possibility of overlooking a lesion in endoscopic examination.

### (Hardware configuration)

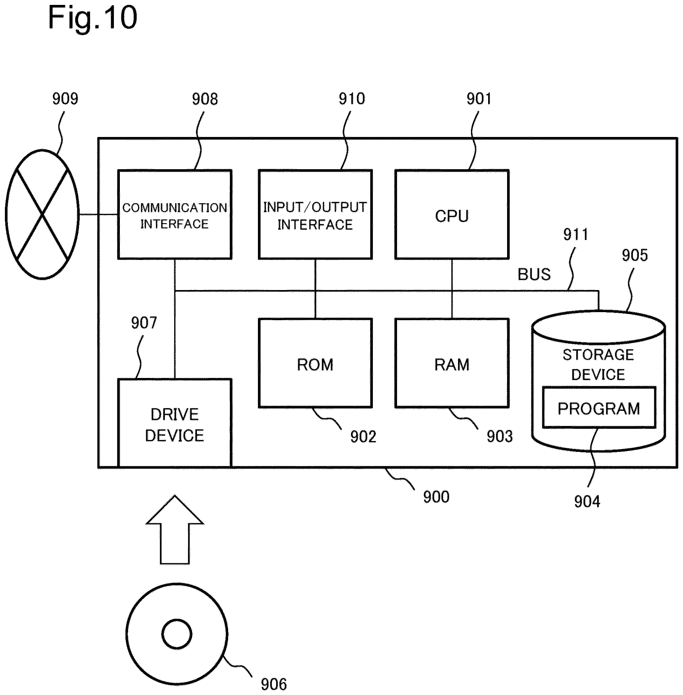

In Figs. 2, 7, and 9 described in the first, second, and third example embodiments, each component of the endoscopic examination assistance device 30, the endoscopic examination assistance device 30a, and the endoscopic examination assistance device 30b is indicated by a block of functional units. Some or all of these components are implemented by an information processing device 900 as illustrated in Fig. 10, for example. Fig. 10 is a block diagram illustrating an example of a hardware configuration of the information processing device 900.

As illustrated in Fig. 10, the information processing device 900 includes the following configuration as an example.

· CPU (Central Processing Unit) 901
· ROM (Read Only Memory) 902
· RAM (Random Access Memory) 903
· Program loaded into RAM 903 904
· Storage device storing program 904 905
· Drive device for reading from and writing to recording medium 906 907
· Communication interface connected to communication network 909 908
· Input/output interface for inputting/outputting data 910
· Bus connecting each component 911

The components of the endoscopic examination assistance device 30, the endoscopic examination assistance device 30a, and the endoscopic examination assistance device 30b described in the first, second, and third example embodiments are implemented by the CPU901 reading and executing the program 904 that implements these functions. The program 904 for achieving the function of each component is stored in the storage device 905 or the ROM 902 in advance, for example, and the CPU 901 loads the program into the RAM 903 and executes the program as necessary. The program 904 may be supplied to the CPU 901 via the communication network 909, or may be stored in advance in the recording medium 906, and the drive device 907 may read the program and supply the program to the CPU 901.

According to the above configuration, the endoscopic examination assistance device 30, the endoscopic examination assistance device 30a, and the endoscopic examination assistance device 30b described in the first, second, and third example embodiments are implemented as hardware. Therefore, an effect similar to the effect described in any one of the example embodiments can be obtained.

Although the present invention has been described with reference to the example embodiments (and examples), the present invention is not limited to the above example embodiments (and examples). It will be understood by those of ordinary skill in the art that various changes in form and details of the example embodiments (and examples) may be made therein without departing from the spirit and scope of the present invention as defined by the claims.

Some or all of the above example embodiments may be described as the following Supplementary Notes, but are not limited to the following.

### [Note 1]

The present invention is not limited to the above-described example embodiments, and various modifications can be made within the scope indicated in the claims. For example, example embodiments obtained by appropriately combining the technical means disclosed in the above-described example embodiments are also included in the technical scope of the present invention.

### [Note 2]

Some or all of the above-described example embodiments can also be described as follows. However, the present invention is not limited to the following aspects.

### (Supplementary Note 1)

An endoscopic examination assistance device including: an acquisition means for acquiring an endoscopic image captured by an imaging unit of an endoscope and imaging unit position information indicating a position of the imaging unit of the endoscope within a lumen at a time when the endoscopic image is captured; a lesion detection means for detecting a lesion from the endoscopic image; a determination means for determining whether it is an insertion step for inserting the endoscope to a turnaround point in the lumen or a removal step for removing the endoscope from a turnaround point in the lumen; and a notification control means for, in a case where the determination result indicates the removal step, causing a notification device to give notification according to a positional relationship between the imaging unit position information and lesion position information indicating a position of a lesion detected in the insertion step.

According to the above configuration, it is possible to reduce the possibility of overlooking a lesion in an endoscopic examination.

### (Supplementary Note 2)

The endoscopic examination assistance device according to Supplementary Note 1, in which, in a case where a distance between a position indicated by the imaging unit position information and a position indicated by the lesion position information is equal to or less than a predetermined distance, the notification control means causes the notification device to give notification that there is a lesion detected in the insertion step nearby a position indicated by the imaging unit position information.

According to the above configuration, a doctor may recognize that there is a lesion detected in the insertion step nearby the imaging unit of the endoscope.

### (Supplementary Note 3)

The endoscopic examination assistance device according to Supplementary Note 1 or 2, in which, in a case where a position indicated by the imaging unit position information has passed a position indicated by the lesion position information, the notification control means causes the notification device to give notification that a lesion detected in the insertion step has been passed.

According to the above configuration, a doctor may recognize that the imaging unit of the endoscope has passed a lesion detected in the insertion step.

### (Supplementary Note 4)

The endoscopic examination assistance device according to any one of Supplementary Notes 1 to 3, in which the notification control means causes the notification device to give notification including an image of the detected lesion, the lesion position information, and a display indicating that the lesion has been detected in the insertion step.

According to the above configuration, a doctor may efficiently recognize information.

### (Supplementary Note 5)

The endoscopic examination assistance device according to any one of Supplementary Notes 1 to 4, in which the imaging unit position information is information indicating a site in the lumen in which the imaging unit is located, the information being generated from a shape of the endoscope in the lumen, a shape of the lumen, and a lumen model indicating each site constituting the lumen.

According to the above configuration, a doctor may efficiently recognize the imaging unit position information.

### (Supplementary Note 6)

The endoscopic examination assistance device according to any one of Supplementary Notes 1 to 5, further including: an identity determination means for determining whether a lesion detected in the removal step is identical to a lesion detected in the insertion step, in which the notification control means causes the notification device to give notification of contents according to a result of the determination.

According to the above configuration, since the notification of the contents according to the determination result as to whether the lesion detected in the insertion step and the lesion detected in the removal step are identical is performed, it is possible to efficiently grasp the examination situation.

### (Supplementary Note 7)

The endoscopic examination assistance device according to Supplementary Note 6, in which, in a case where a position indicated by the imaging unit position information has passed a position indicated by the lesion position information without determining that a lesion detected in the removal step is identical to a lesion detected in the insertion step, the notification control means causes the notification device to give notification that a lesion detected in the insertion step has been passed without being detected.

According to the above configuration, a doctor may return (move in the insertion direction) the endoscope to look for the lesion, and the possibility of overlooking a lesion can be reduced.

### (Supplementary Note 8)

An endoscopic examination assistance system including: an endoscopic examination assistance device; and a notification device, in which the endoscopic examination assistance device includes: an acquisition means for acquiring an endoscopic image captured by an imaging unit of an endoscope and imaging unit position information indicating a position of the imaging unit of the endoscope within a lumen at a time when the endoscopic image is captured; a lesion detection means for detecting a lesion from the endoscopic image; a determination means for determining whether it is an insertion step for inserting the endoscope to a turnaround point in the lumen or a removal step for removing the endoscope from a turnaround point in the lumen; and a notification control means for, in a case where the determination result indicates the removal step, causing the notification device to give notification according to a positional relationship between the imaging unit position information and lesion position information indicating a position of a lesion detected in the insertion step.

According to the above configuration, it is possible to reduce the possibility of overlooking a lesion in an endoscopic examination.

### (Supplementary Note 9)

An endoscopic examination assistance method causing one or a plurality of endoscopic examination assistance devices to execute: acquiring an endoscopic image captured by an imaging unit of an endoscope and imaging unit position information indicating a position of the imaging unit of the endoscope within a lumen at a time when the endoscopic image is captured; detecting a lesion from the endoscopic image; determining whether it is an insertion step for inserting the endoscope to a turnaround point in the lumen or a removal step for removing the endoscope from a turnaround point in the lumen; and in a case where the determination result indicates the removal step, causing a notification device to give notification according to a positional relationship between the imaging unit position information and lesion position information indicating a position of a lesion detected in the insertion step.

According to the above method, it is possible to reduce the possibility of overlooking a lesion in the endoscopic examination.

### (Supplementary Note 10)

A recording medium having stored therein a program causing a computer to function as: an acquisition means for acquiring an endoscopic image captured by an imaging unit of an endoscope and imaging unit position information indicating a position of the imaging unit of the endoscope within a lumen at a time when the endoscopic image is captured; a lesion detection means for detecting a lesion from the endoscopic image; a determination means for determining whether it is an insertion step for inserting the endoscope to a turnaround point in the lumen or a removal step for removing the endoscope from a turnaround point in the lumen; and a notification control means for, in a case where the determination result indicates the removal step, causing a notification device to give notification according to a positional relationship between the imaging unit position information and lesion position information indicating a position of a lesion detected in the insertion step.

According to the program or the recording medium storing the program, it is possible to reduce the possibility of overlooking a lesion in endoscopic examination.

### [Note 3]

Some or all of the above-described example embodiments can be further expressed as follows.

An endoscopic examination assistance device including at least one processor, the processor executing: a process of acquiring an image captured by an imaging unit of an endoscope and imaging unit position information indicating a position of the imaging unit of the endoscope within a lumen at a time when the image is captured; a process of detecting a lesion from the image; a process of determining whether it is an insertion step for inserting the endoscope to a turnaround point in the lumen or a removal step for removing the endoscope from a turnaround point in the lumen; and a process of, in a case where the determination result indicates the removal step, causing a notification device to give notification according to a positional relationship between the imaging unit position information and lesion position information indicating a position of a lesion detected in the insertion step.

This endoscopic examination assistance device may further include a memory having stored therein an endoscopic examination assistance program causing a processor to execute: acquiring an endoscopic image captured by an imaging unit of an endoscope and imaging unit position information indicating a position of the imaging unit of the endoscope within a lumen at a time when the endoscopic image is captured; detecting a lesion from the endoscopic image; determining whether it is an insertion step for inserting the endoscope to a turnaround point in the lumen or a removal step for removing the endoscope from a turnaround point in the lumen; and causing, in a case where the determination result indicates the removal step, a notification device to give notification according to a positional relationship between the imaging unit position information and lesion position information indicating a position of a lesion detected in the insertion step. The endoscopic examination assistance program may be recorded in a non-transitory tangible computer-readable recording medium.

### Reference Signs List

- 1: endoscopic examination assistance system
- 10: endoscope
- 12: imaging unit
- 20: endoscope insertion shape observation device
- 21: sense coil unit
- 22: position information processing device
- 30, 30a, 30b: endoscopic examination assistance device
- 31, 31b: acquisition unit
- 32, 32b: lesion detection unit
- 33, 33b: determination unit
- 34: storage unit
- 35, 35a, 34b: notification control unit
- 36: ileocecal valve detection unit
- 37: identity determination unit
- 41: display device
- 42: speaker
- 901: CPU (Central Processing Unit)
- 902: ROM (Read Only Memory)
- 903: RAM (Random Access Memory)
- 904: program
- 905: storage device
- 906: recording medium
- 907: drive device
- 908: communication interface
- 909: communication network
- 910: input/output interface
- 911: bus

## Claims

1. An endoscopic examination assistance device comprising:
an acquisition means for acquiring an endoscopic image captured by an imaging unit of an endoscope and imaging unit position information indicating a position of the imaging unit of the endoscope within a lumen at a time when the endoscopic image is captured;
a lesion detection means for detecting a lesion from the endoscopic image;
a determination means for determining whether it is an insertion step for inserting the endoscope to a turnaround point in the lumen or a removal step for removing the endoscope from a turnaround point in the lumen; and
a notification control means for, in a case where a result of the determination indicates the removal step, causing a notification device to give notification according to a positional relationship between the imaging unit position information and lesion position information indicating a position of a lesion detected in the insertion step.

2. The endoscopic examination assistance device according to claim 1, wherein
in a case where a distance between a position indicated by the imaging unit position information and a position indicated by the lesion position information is equal to or less than a predetermined distance, the notification control means causes the notification device to give notification that there is a lesion detected in the insertion step nearby a position indicated by the imaging unit position information.

3. The endoscopic examination assistance device according to claim 1 or 2, wherein
in a case where a position indicated by the imaging unit position information has passed a position indicated by the lesion position information, the notification control means causes the notification device to give notification that a lesion detected in the insertion step has been passed.

4. The endoscopic examination assistance device according to any one of claims 1 to 3, wherein
the notification control means causes the notification device to give notification including an image of the detected lesion, the lesion position information, and a display indicating that the lesion has been detected in the insertion step.

5. The endoscopic examination assistance device according to any one of claims 1 to 4, wherein
the imaging unit position information is information indicating a site in the lumen in which the imaging unit is located, the information being generated from a shape of the endoscope in the lumen, a shape of the lumen, and a lumen model indicating each site constituting the lumen.

6. The endoscopic examination assistance device according to any one of claims 1 to 5, further comprising:
an identity determination means for determining whether a lesion detected in the removal step is identical to a lesion detected in the insertion step, wherein
the notification control means causes the notification device to give notification of contents according to a result of the determination.

7. The endoscopic examination assistance device according to claim 6, wherein
in a case where a position indicated by the imaging unit position information has passed a position indicated by the lesion position information without determining that a lesion detected in the removal step is identical to a lesion detected in the insertion step, the notification control means causes the notification device to give notification that a lesion detected in the insertion step has been passed without being detected.

8. An endoscopic examination assistance system comprising: an endoscopic examination assistance device; and a notification device, wherein
the endoscopic examination assistance device includes:
an acquisition means for acquiring an endoscopic image captured by an imaging unit of an endoscope and imaging unit position information indicating a position of the imaging unit of the endoscope within a lumen at a time when the endoscopic image is captured;
a lesion detection means for detecting a lesion from the endoscopic image;
a determination means for determining whether it is an insertion step for inserting the endoscope to a turnaround point in the lumen or a removal step for removing the endoscope from a turnaround point in the lumen; and
a notification control means for, in a case where a result of the determination indicates the removal step, causing the notification device to give notification according to a positional relationship between the imaging unit position information and lesion position information indicating a position of a lesion detected in the insertion step.

9. An endoscopic examination assistance method causing one or a plurality of endoscopic examination assistance devices to execute:
acquiring an endoscopic image captured by an imaging unit of an endoscope and imaging unit position information indicating a position of the imaging unit of the endoscope within a lumen at a time when the endoscopic image is captured;
detecting a lesion from the endoscopic image;
determining whether it is an insertion step for inserting the endoscope to a turnaround point in the lumen or a removal step for removing the endoscope from a turnaround point in the lumen; and
in a case where a result of the determination indicates the removal step, causing a notification device to give notification according to a positional relationship between the imaging unit position information and lesion position information indicating a position of a lesion detected in the insertion step.

10. A recording medium having stored therein a program causing a computer to function as:
an acquisition means for acquiring an endoscopic image captured by an imaging unit of an endoscope and imaging unit position information indicating a position of the imaging unit of the endoscope within a lumen at a time when the endoscopic image is captured;
a lesion detection means for detecting a lesion from the endoscopic image;
a determination means for determining whether it is an insertion step for inserting the endoscope to a turnaround point in the lumen or a removal step for removing the endoscope from a turnaround point in the lumen; and
a notification control means for, in a case where a result of the determination indicates the removal step, causing a notification device to give notification according to a positional relationship between the imaging unit position information and lesion position information indicating a position of a lesion detected in the insertion step.
